# FASCICULE DE BREVET EUROPEEN

(11) **EP 0 953 335 B1**
(45) Date de publication et mention de la délivrance du brevet: **09.01.2002**
(21) Numéro de dépôt: 99400630.2
(22) Date de dépôt: 15.03.1999
(51) Int. Cl.: A61K 7/32

(54) **Composition solide déodorante**
Feste Deodorantien
Solid deodorant composition

(30) Priorité: 23.03.1998 FR 9803539
(43) Date de publication de la demande: 03.11.1999
(73) Titulaire: L'OREAL, 75008 Paris (FR)
(72) Inventeur: Pataut, Françoise, 75017 Paris (FR); Aubert, Lionel, 95330 Domont (FR)
(74) Mandataire: Casalonga, Axel

(56) Documents cités:
- JP-A- 57 112 315
- US-A- 4 822 603
- CHEMICAL ABSTRACTS, vol. 104, no. 2, 13 janvier 1986 (1986-01-13) Columbus, Ohio, US; abstract no. 10407, CHINNO, TOSHIHIRO ET AL: "Antiperspirant sticks" XP002090720 & JP 60 181011 A (LION CORP., JAPAN)
- DATABASE WPI Week 198233, Derwent Publications Ltd., London, GB; Class A96, AN 1982-69438E & JP 57 112 315 A (LION CORP) 13 Juillet 1982

## Description

Les sticks déodorants anhydres sont connus et utilisés depuis de nombreuses années. Ils offrent de nombreux avantages par rapport à d'autres formes de présentation de déodorants, en particulier par rapport aux aérosols. Ces avantages sont par exemple une grande sécurité d'utilisation et de stockage, l'absence de gaz propulseurs nocifs pour la couche d'ozone, un emballage simple et économique, une bonne stabilité à la conservation due à l'absence de phénomènes de dessèchement.

On utilise traditionnellement comme agent solidifiant dans les compositions déodorantes anhydres une association de deux types de cires, l'une ayant un point de fusion relativement bas, à savoir inférieur à 80 °C, et l'autre ayant un point de fusion élevé c'est-à-dire supérieur à 80 °C. La cire à bas point de fusion la plus couramment décrite dans la littérature et utilisée dans les produits commercialisés est l'alcool stéarylique qui a un point de fusion de 60 °C.

L'association d'une cire à haut point de fusion et d'une cire à bas point de fusion telle que l'alcool stéarylique a pour principaux inconvénients la fragilité du pain des sticks réduisant leur durabilité et le dépôt, sur la peau, d'importantes traces blanchâtres, susceptibles d'être ensuite transférées sur les vêtements.

La demande de brevet japonais JP 57 - 112315 divulgue des sticks déodorants, sous forme de poudre compacte, contenant une cire naturelle et de 60 à 95 % de charge pulvérulente.

La demanderesse a découvert à présent qu'il était possible de surmonter ces inconvénients en utilisant, comme agent solidifiant de compositions déodorantes anhydres, un mélange de cires exempt de cires à bas point de fusion, c'est-à-dire un mélange composé exclusivement de cires ayant toutes un point de fusion supérieur à 80 °C.

Les compositions déodorantes de la présente invention, solidifiées par une telle association d'au moins deux cires à haut point de fusion, présentent une excellente durabilité à l'utilisation et un moindre blanchiment de la peau après application tout en conservant leurs qualités cosmétiques, notamment les bonnes propriétés à l'étalement telles que l'onctuosité, le glissant et l'usure.

La présente invention a par conséquent pour objet une composition solide déodorante selon la revendication 1.

La ou les cires polyéthyléniques ont pour fonction de conférer au produit ses propriétés nécessaires d'étalement, d'usure et de glisse. La ou les cires naturelles à point de fusion élevé, en association avec la ou les premières cires, renforcent la structure du pain du stick et apportent la rigidité nécessaire pour éviter l'effritement des sticks.

La cire polyéthylénique à haut point de fusion (> 80 °C) utilisée selon l'invention est un homopolymère d'éthylène ou un copolymère d'éthylène et d'un autre monomère copolymérisable répondant à la formule (I) suivante

CH₂=CHR (I)

dans laquelle R représente une chaîne alkyle linéaire ou ramifiée pouvant être interrompue par des motifs mono- ou polyoxyalkylènes, un radical aryle ou aralkyle, un radical -CH₂COOH ou -CH₂CH₂OH.

Les radicaux alkyle désignent plus particulièrement les radicaux méthyle, éthyle, propyle, isopropyle, décyle, dodécyle et octadécyle,

Les motifs mono- ou polyoxyalkylènes désignent de préférence des groupes mono- ou polyoxyéthylène ou des groupes mono- ou polyoxypropylène. Le radical aryle est de préférence un radical phényle ou tolyle.

Le radical aralkyle est par exemple un radical benzyle ou phénéthyle.

La masse molaire moyenne en poids de la cire polyéthylénique à haut point de fusion selon l'invention est de préférence comprise entre environ 400 et 1000, plus particulièrement entre environ 400 et 700 et est de préférence d'environ 500.

Selon un mode de réalisation préféré des compositions selon l'invention, la cire telle que définie précédemment est choisie parmi les homopolymères d'éthylène, les copolymères d'éthylène et de propylène, les copolymères d'éthylène et d'anhydride ou d'acide maléique, les polyéthylènes oxydés ou éthoxylés.

Parmi les homopolymères d'éthylène utilisables selon l'invention, on peut citer notamment ceux commercialisés sous les dénominations de POLYWAX® 500, POLYWAX® 655 et POLYWAX® 1000 par la société PETROLITE.

Parmi les copolymères d'éthylène utilisables selon l'invention, on peut citer les copolymères d'éthylène et de propylène commercialisés sous les dénominations PETROLITE® par la société PETROLITE, les copolymères d'éthylène et d'anhydride maléique commercialisés sous les dénominations CERAMER® par la société PETROLITE, les polyéthylènes oxydés commercialisés sous les dénominations UNILIN® et UNICID® par la société PETROLITE, et les polyéthylènes éthoxylés commercialisés sous les dénominations UNITHOX® par la société PETROLITE.

Selon un mode de réalisation particulièrement préféré de l'invention, la cire polyéthylénique est une cire d'homopolymère d'éthylène.

Dans les compositions déodorantes anhydres de la présente invention, la cire polyéthylénique décrite ci-dessus est associée à au moins une deuxième cire qui est une cire naturelle ayant également un point de fusion supérieur à 80 °C. Cette cire naturelle est choisie parmi les cires minérales, fossiles, animales ou végétales, ou les huiles hydrogénées, les esters gras, les alcools gras ou les alcools gras polyoxyéthylénés concrèts à 25 °C.

On peut utiliser, à titre d'exemples de cires d'origine naturelle dans les compositions déodorantes de la présente invention, les cires microcristallines, la cérésine, l'ozokérite, la cire de candelilla, la cire de carnauba, l'huile de ricin hydrogénée, l'huile de palme hydrogénée, l'huile de coco hydrogénée.

Selon un mode de réalisation préféré de l'invention, la cire naturelle est une ozokérite à haut point de fusion. L'ozokérite est un hydrocarbure fossile de composition complexe correspondant au résidu solide d'évaporation de pétroles riches en paraffines.

Une ozokérite commerciale est le produit CEROZO BLANCHE E 626®, un mélange d'hydrocarbures en C₂₀₋₅₀ commercialisé par la société BARLOCHER.

La composition déodorante de la présente invention contient de 5 à 20 % en poids de cire synthétique polyéthylénique et de 2 à 20 % en poids de ladite cire naturelle à haut point de fusion.

La proportion de cire polyéthylénique est de préférence supérieure à celle de la cire naturelle et le rapport pondéral (cire polyéthylénique/cire naturelle) est compris notamment entre 10/1 et 1/1 et en particulier entre 7/1 et 3/1.

La composition déodorante de la présente invention contient, en plus du mélange de cires à haut point de fusion, au moins un actif déodorant.

Au sens de la présente invention, on entend par actif déodorant toute substance capable de réduire le flux sudoral et/ou masquer, améliorer ou réduire l'odeur désagréable résultant de la décomposition de la sueur humaine par des bactéries.

Il s'agit par exemple d'un composé anti-transpirant tel que les sels d'aluminium et/ou de zirconium, par exemple un chlorhydrate d'aluminium ou un chlorhydrate d'aluminium et de zirconium, les sels d'alun, d'un agent bactériostatique, d'un agent bactéricide tel que le 2,4,4'-trichloro-2'-hydroxydiphényléther et le 3,7,11-triméthyldodéca-2,5,10-triénol, et différents sels de zinc, d'un agent absorbant les odeurs tel que le bicarbonate de sodium, d'un agent antioxydant tel que le butylhydroxytoluène ou d'un mélange de ces composés et/ou agents. Le 3,7,11-triméthyldodéca-2,5,10-triénol est par exemple vendu sous la dénomination FARNESOL® par la société DRAGOCO et le 2,4,4'-trichloro-2'-hydroxydiphényléther sous la dénomination IRGACARE® MP par la société CIBA-GEIGY.

Parmi les sels d'aluminium, on peut citer le produit commercialisé par la société REHEIS sous la dénomination REACH® 301 ou par la société GUILINI CHEMIE sous la dénomination ALOXICOLL® PF 40.

Des sels d'aluminium et de zirconium sont par exemple celui commercialisé par la société REHEIS sous la dénomination REACH® A2P-908-SUF.

Cet actif déodorant ou mélange d'actifs déodorants est présent à raison d'environ 0,01 à 40 % en poids rapporté à la composition totale, de préférence à raison d'environ 0,05 à 25 % en poids.

Lorsqu'il s'agit d'un chlorhydrate d'aluminium ou d'un chlorhydrate d'aluminium et de zirconium, il est présent notamment à raison de 2 à 30 % en poids, de préférence à raison de 5 à 25 % en poids, par rapport au poids total de la composition.

La composition solide déodorante de la présent invention peut contenir en outre un ou plusieurs agents émollients. Ces agents émollients ont pour fonction d'améliorer les propriétés cosmétiques du produit final, de favoriser un bon glissement du pain du stick sur la peau et de faciliter ainsi l'application de la composition déodorante. Les agents émollients sont par exemple des silicones volatiles telles que le produit commmercialisé sous la dénomination DC246 Fluid par la société DOW CORNING, et des silicones non volatiles comme par exemple le produit DC 556 commercialisé par la société DOW CORNING, des polydécènes tels que le produit SILKFLO® S366 NF de la société AMOCO, des esters d'acides gras tels que le produit commercialisé par la société UNICHEMA sous la dénomination ESTOL® 1517, des alcools gras, des agents tensio-actifs éthoxylés et/ou propoxylés ou des esters de glycol polyalcoxylés ou du PPG(14) butyléther (Fluide AP de la société UNION CARBIDE).

La teneur en agents émollients de la composition de la présente invention est généralement comprise entre 10 et 70 % en poids, de préférence entre 20 et 60 % en poids.

La composition déodorante selon l'invention contient en outre généralement une ou plusieurs charges incorporée(s) sous forme de particules très fines dans la matière grasse de la composition. Ces chargés sont choisies par exemple parmi le talc, la silice, les dérivés de l'amidon, l'argile ou des copolymères d'acrylates (EXPANSEL® 551 DE de la société KEMANORD) et sont généralement présentes à raison d'environ 0,1 à 10 % en poids rapporté à la masse totale de la composition.

La composition déodorante selon l'invention peut contenir en outre d'autres adjuvants tels que par exemple des parfums et/ou des colorants. Bien entendu, l'homme de métier veillera à choisir ce ou ces éventuels composés complémentaires de manière telle que les propriétés avantageuses attachées intrinsèquement à la composition déodorante conforme à l'invention ne soient pas, ou substantiellement pas, altérées par la ou les adjonctions envisagées.

La composition solide déodorante de l'invention se présente généralement sous forme d'un stick déodorant.

La présente invention a également pour objet l'utilisation des compositions décrites ci-dessus pour réduire le flux sudoral et/ou masquer, améliorer ou réduire l'odeur désagréable résultant de la décomposition de la sueur humaine par des bactéries.

Cette utilisation consiste à appliquer de préférence quotidiennement la composition déodorante solide sur les parties de peau propre que l'on souhaite traiter, de préférence sous les aisselles.

Les exemples ci-dessous, non limitatifs, illustrent la présente invention.

### Exemples 1 et 2 et exemples comparatifs A et B

On prépare quatre compositions solides déodorantes 1,2, A et B présentées dans le Tableau 1 ci-dessous et qui diffèrent essentiellement par la nature des agents solidifiants utilisés.

Les compositions 1 et 2 sont des compositions conformes à la présente invention contenant, comme agent solidifiant, deux cires à point de fusion supérieur à 80 °C. Les compositions A et B sont des compositions correspondant à l'état de la technique et comprenant, comme agent solidifiant, une cire à haut point de fusion (> 80 °C) et une cire à bas point de fusion (< 80 °C), à savoir l'alcool stéarylique qui a un point de fusion de 60 °C.

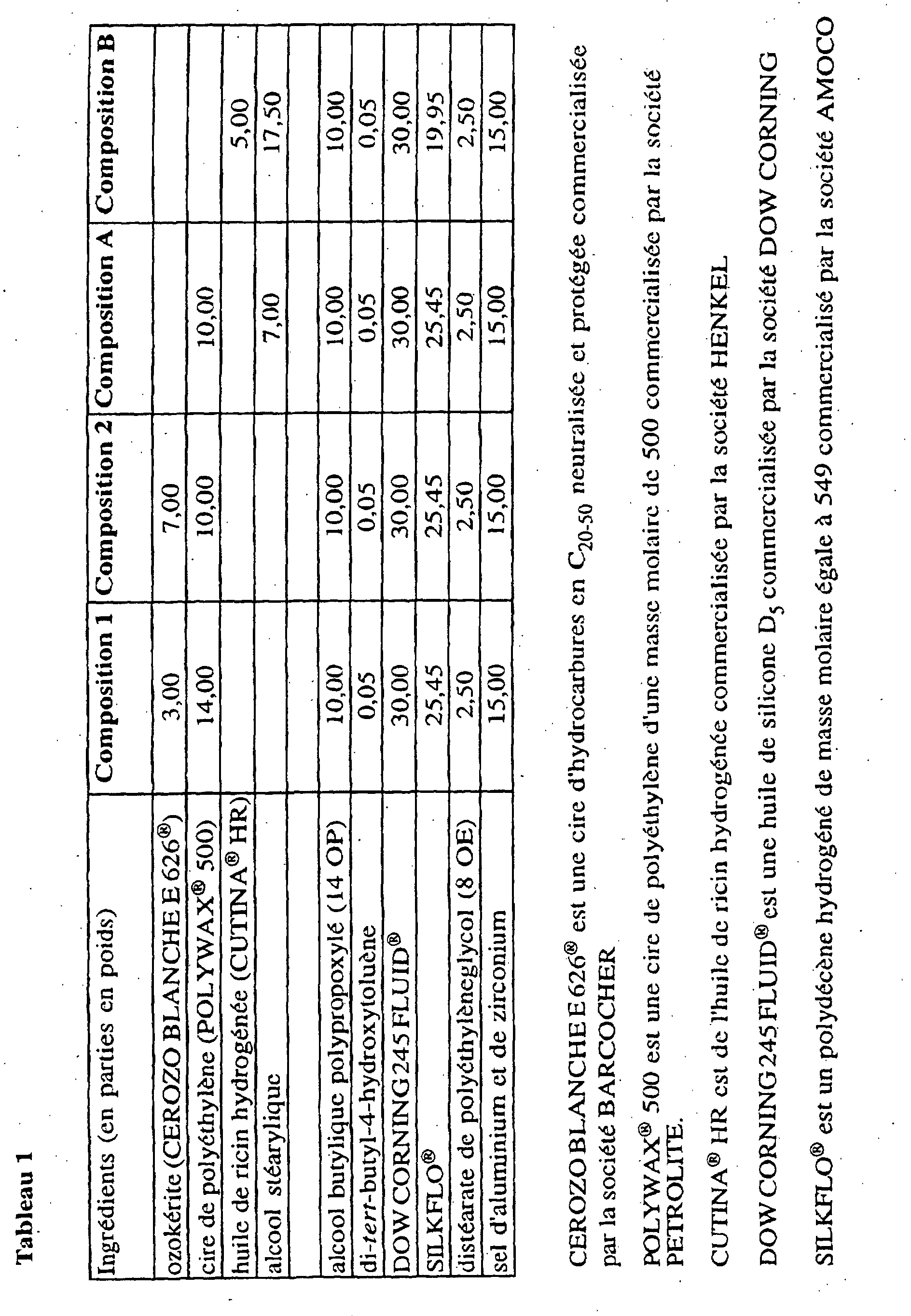

Chacune de ces quatre compositions déodorantes est soumise à deux essais destinés à évaluer :
1- leur résistance au cisaillement
2- leur fragilité statique.

1- L'*essai de cisaillement* consiste à mesurer la force maximale (en N), exercée par un fil normalisé, que supporte un échantillon dans des conditions définies avant d'être tranché par ce fil. Cet essai est effectué au moyen d'un ruptomètre électronique conçu par l'ORÉAL RAD et fabriqué par la société J. BOULEFFROY (Estrées Saint-Denis, France).

L'échantillon à tester est placé pendant au moins 16 heures dans une enceinte réglée et stabilisée à 20 ± 1 °C. L'échantillon est fixé, immédiatement avant la mesure, dans le porte-godet réglé de façon à ce que l'écartement entre le fil et le raisin du stick soit égal à 2 - 3 mm et que le fil soit situé perpendiculairement à l'axe longitudinal du stick à exactement 10 mm de la base (godet) de celui-ci. On mesure ensuite la force minimale (en N) nécessaire pour trancher le stick avec ledit fil normalisé.

2- L'*essai de fragilité statique* consiste à mesurer la force maximale (en N), exercée par un outil normalisé (surface plane d'un étrier), que supporte un échantillon dans des conditions définies avant d'être cassé par cet outil. Cet essai est également réalisé à l'aide du ruptomètre ayant servi pour la mesure de la résistance au cisaillement décrite ci-dessus.

L'échantillon à tester est placé pendant au moins 16 heures dans une enceinte réglée et stabilisée à 20 ± 1 °C. Il est fixé, immédiatement avant la mesure, dans le porte-godet réglé de façon à de ce que le pan coupé du stick soit parallèle à la surface plane de l'étrier à une distance de 3 mm de celui-ci. On mesure ensuite la force minimale (en N) nécessaire pour que la surface plane de l'étrier, pressée contre le pan coupé du stick, casse celui-ci.

Les résultats de ces essais obtenus pour les deux compositions de l'invention (1 et 2) et les deux compositions comparatives (A et B) sont indiqués dans le Tableau 2 suivant.

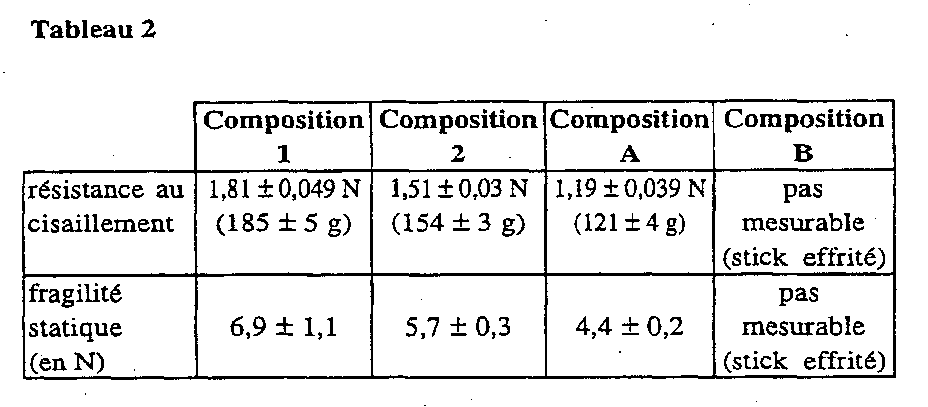

Les valeurs obtenues sont les moyennes arithmétiques ± l'écart type, calculés à partir de 10 mesures.

Ces résultats montrent que les Compositions 1 et 2 selon l'invention présentent une résistance au cisaillement et une fragilité statique significativement meilleures que celles des Compositions A et B, dont la dernière est même trop fragile pour permettre des mesures.

## Revendications

1. Composition solide déodorante essentiellement anhydre, comprenant de 0,01 % à 40 % en poids d'au moins un actif déodorant et, comme agent solidifiant, un mélange de cires ayant toutes un point de fusion supérieur à 80 °C comprenant :
- au moins une cire synthétique polyéthylénique ayant un point de fusion supérieur à 80 °C, et
- au moins une cire d'origine naturelle ayant un point de fusion supérieur à 80 °C.

2. Composition solide déodorante selon la revendication 1, **caractérisée en ce que** la cire synthétique à point de fusion supérieur à 80 °C est un homopolymère d'éthylène ou un copolymère d'éthylène et d'un autre monomère copolymérisable répondant à la formule
CH₂=CH-R (I)
dans laquelle R représente une chaîne alkyle linéaire ou ramifiée pouvant être interrompu par des motifs mono- ou polyoxyalkylènes, un radical aryle ou aralkyle, un radical -CH₂COOH ou -CH₂CH₂OH.

3. Composition solide déodorante selon la revendication 2, **caractérisée en ce que** la cire homopolymère ou copolymère d'éthylène a une masse molaire moyenne en poids comprise entre 400 et 1000.

4. Composition solide déodorante selon la revendication 3, **caractérisée par le fait que** ladite cire est un homopolymère d'éthylène ayant une masse molaire d'environ 500 .

5. Composition solide déodorante selon l'une quelconque des revendications 1 à 4, **caractérisée par le fait que** la cire naturelle ayant un point de fusion supérieur à 80 °C est choisie parmi les cires minérales, fossiles, animales ou végétales, ou les huiles hydrogénées, esters gras, les alcools gras ou les alcools gras polyalcoxylés.

6. Composition solide déodorante selon la revendication 5, **caractérisée par le fait que** ladite cire naturelle est choisie parmi les cires microcristallines, la cérésine, l'ozokérite, la cire de candellila, la cire de carnauba, l'huile de ricin hydrogénée, l'huile de palme hydrogénée, l'huile de coco hydrogénée.

7. Composition solide déodorante selon la revendication 6, **caractérisée par le fait que** la cire naturelle est l'ozokérite.

8. Composition solide déodorante selon l'une quelconque des revendications 1 à 7, **caractérisé par le fait que** ladite cire à base de polyéthylène est présente à raison de 5 à 20 % en poids par rapport à la masse totale de la composition.

9. Composition solide déodorante selon l'une quelconque des revendications 1 à 8, **caractérisée par le fait que** ladite cire naturelle est présente à raison de 2 à 20 % en poids par rapport à la masse totale de la composition.

10. Composition solide déodorante selon la revendication 8 ou 9, **caractérisée par le fait que** ladite cire synthétique polyéthylénique est présente en une proportion supérieure à celle de ladite cire naturelle.

11. Composition solide déodorante selon la revendication 8 ou 9, **caractérisée par le fait que** le rapport pondéral de ladite cire synthétique à ladite cire naturelle est compris entre 10/1 et 1/1, de préférence entre 7/1 et 3/1.

12. Composition solide déodorante selon l'une quelconque des revendications 1 à 11, **caractérisée en ce que** l'actif déodorant est présent à à raison de 0,05 à 25 % en poids par rapport à la masse totale de la composition.

13. Composition solide déodorante selon l'une quelconque des revendications 1 à 12, **caractérisée par le fait que** l'actif déodorant est choisi parmi les sels d'aluminium et/ou les sels de zirconium, les sels d'alun, les agents bactériostatiques, les agents bactéricides, les substances absorbant les odeurs et les agents antioxydants.

14. Composition solide déodorante selon la revendication 13, **caractérisée par le fait que** l'actif déodorant est un chlorhydrate d'aluminium ou un chlorhydrate d'aluminium et de zirconium.

15. Composition solide déodorante selon la revendication 14, **caractérisée par le fait que** le chlorhydrate d'aluminium ou le chlorhydrate d'aluminium et de zirconium est présent à raison de 2 à 30 % en poids, de préférence à raison de 5 à 25 % en poids, par rapport à la masse totale de la composition.

16. Composition solide déodorante selon l'une quelconque des revendications 1 à 15, **caractérisée en ce qu'**elle contient en outre un ou plusieurs agents émollients.

17. Composition solide déodorante selon la revendication 16, **caractérisée par le fait que** le ou les agents émollients sont choisis parmi les silicones volatiles et non volatiles, les polydécènes, les esters d'acides gras, les alcools gras, les agents tensioactifs éthoxylés et/ou propoxylés, les esters de glycol polyalcoxylés ou du polypropylèneglycol(14)butyléther.

18. Composition solide déodorante selon la revendication 17, **caractérisée par le fait que** le ou les agents émollients sont présents à raison de 10 à 70 % en poids par rapport à la masse totale de la composition.

19. Composition solide déodorante selon l'une quelconque des revendications 1 à 18, **caractérisée par le fait qu'**elle contient en outre une ou plusieurs charges.

20. Composition solide déodorante selon la revendication 19, **caractérisée par le fait que** la ou les charges sont choisies parmi le talc, la silice, les dérivés d'amidon, l'argile ou des copolymères d'acrylates.

21. Composition solide déodorante selon la revendication 20, **caractérisée par le fait que** la ou les charges sont présentes à raison de 0,1 à 10 % en poids par rapport à la masse totale de la composition.

22. Composition solide déodorante selon l'une quelconque des revendications 1 à 21, **caractérisée par le fait qu'**elle contient en outre d'autres adjuvants tels que des parfums ou des colorants.

23. Composition solide déodorante selon l'une quelconque des revendications 1 à 22, **caractérisée par le fait qu'**elle se présente sous forme d'un stick.

24. Utilisation cosmétique d'une composition solide selon l'une quelconque des revendications 1 à 23, pour réduire le flux sudoral et/ou masquer, améliorer ou réduire l'odeur désagréable résultant de la décomposition de la sueur humaine par des bactéries.

## Patentansprüche

1. Im wesentlichen wasserfreie desodorierende feste Zusammensetzung, die 0,01 bis 40 Gew.-% mindestens eines desodorierenden Wirkstoffes und als Verfestiger ein Gemisch von Wachsen enthält, die alle einen Schmelzpunkt über 80 °C aufweisen und umfassen:
- mindestens ein synthetisches Polyethylenwachs mit einem Schmelzpunkt über 80 °C, und
- mindestens ein Wachs natürlicher Herkunft mit einem Schmelzpunkt über 80 °C.

2. Desodorierende feste Zusammensetzung nach Anspruch 1, **dadurch gekennzeichnet, daß** das synthetische Wachs mit einem Schmelzpunkt über 80 °C ein Homopolymer von Ethylen oder ein Copolymer von Ethylen und einem weiteren copolymerisierbaren Monomer der folgenden Formel (I) ist:
CH₂=CH-R (I),
worin R eine geradkettige oder verzweigte Alkylgruppe, die durch Mono- oder Polyoxyalkylengruppen unterbrochen sein kann, eine Aryl- oder Aralkylgruppe, eine Gruppe -CH₂COOH oder eine Gruppe -CH₂CH₂OH bedeutet.

3. Desodorierende feste Zusammensetzung nach Anspruch 2, **dadurch gekennzeichnet, daß** das als Wachs vorliegende Homopolymer oder Copolymer von Ethylen ein Gewichtsmittel des Molekulargewichts von 400 bis 1000 aufweist.

4. Desodorierende feste Zusammensetzung nach Anspruch 3, **dadurch gekennzeichnet, daß** das Wachs ein Ethylenhomopolymer mit einem Molekulargewicht von etwa 500 ist.

5. Desodorierende feste Zusammensetzung nach einem der Ansprüche 1 bis 4, **dadurch gekennzeichnet, daß** das natürliche Wachs mit einem Schmelzpunkt über 80 °C unter den mineralischen, fossilen, tierischen oder pflanzlichen Wachsen, hydrierten Ölen, Fettsäureestern, Fettalkoholen oder polyalkoxylierten Fettalkoholen ausgewählt ist.

6. Desodorierende feste Zusammensetzung nach Anspruch 5, **dadurch gekennzeichnet, daß** das natürliche Wachs unter den mikrokristallinen Wachsen, Ceresin, Ozokerit, Candelillawachs, Carnaubawachs, hydriertem Ricinusöl, hydriertem Palmöl und hydriertem Cocosöl ausgewählt ist.

7. Desodorierende feste Zusammensetzung nach Anspruch 6, **dadurch gekennzeichnet, daß** es sich bei dem natürlichen Wachs um Ozokerit handelt.

8. Desodorierende feste Zusammensetzung nach einem der Ansprüche 1 bis 7, **dadurch gekennzeichnet, daß** das Wachs auf Polyethylenbasis in Mengenanteilen von 5 bis 20 Gew.-%, bezogen auf das Gesamtgewicht der Zusammensetzung, vorliegt.

9. Desodorierende feste Zusammensetzung nach einem der Ansprüche 1 bis 8, **dadurch gekennzeichnet, daß** das natürliche Wachs in einem Mengenanteil von 2 bis 20 Gew.-%, bezogen auf das Gesamtgewicht der Zusammensetzung, vorliegt.

10. Desodorierende feste Zusammensetzung nach Anspruch 8 oder 9, **dadurch gekennzeichnet, daß** das synthetische Polyethylenwachs in einem Mengenanteil vorliegt, der über dem Mengenanteil des natürlichen Wachses liegt.

11. Desodorierende feste Zusammensetzung nach Anspruch 8 oder 9, **dadurch gekennzeichnet, daß** das Gewichtsverhältnis des synthetischen Wachses und des natürlichen Wachses im Bereich von 10/1 bis 1/1 und vorzugsweise 7/1 bis 3/1 liegt.

12. Desodorierende feste Zusammensetzung nach einem der Ansprüche 1 bis 11, **dadurch gekennzeichnet, daß** der desodorierende Wirkstoff in einer Menge von 0,05 bis 25 Gew.-%, bezogen auf das Gesamtgewicht der Zusammensetzung, vorliegt.

13. Desodorierende feste Zusammensetzung nach einem der Ansprüche 1 bis 12, **dadurch gekennzeichnet, daß** der desodorierende Wirkstoff unter den Aluminiumsalzen und/oder Zirconiumsalzen, Alaunsalzen, bakteriostatischen Mitteln, bakteriziden Mitteln, Geruchsabsorbern und Antioxidantien ausgewählt ist.

14. Desodorierende feste Zusammensetzung nach Anspruch 13, **dadurch gekennzeichnet, daß** der desodorierende Wirkstoff ein Aluminiumhydroxychlorid oder ein Hydroxychlorid von Aluminium und Zirconium ist.

15. Desodorierende feste Zusammensetzung nach Anspruch 14, **dadurch gekennzeichnet, daß** das Aluminiumhydroxychlorid oder Hydroxychlorid von Aluminium und Zirconium in Mengenanteilen von 2 bis 30 Gew.-% und vorzugsweise 5 bis 25 Gew.-%, bezogen auf das Gesamtgewicht der Zusammensetzung, vorliegt.

16. Desodorierende feste Zusammensetzung nach einem der Ansprüche 1 bis 15, **dadurch gekennzeichnet, daß** sie ferner ein Emolliens oder mehrere Emollientien enthält.

17. Desodorierende feste Zusammensetzung nach Anspruch 16, **dadurch gekennzeichnet, daß** das Emolliens oder die Emollientien unter den flüchtigen und nicht flüchtigen Siliconen, Polydecenen, Fettsäureestern, Fettalkoholen, ethoxylierten und/oder propoxylierten grenzflächenaktiven Stoffen, polyalkoxylierten Glykolestern und Polypropylenglykol(14)butylether ausgewählt sind.

18. Desodorierende feste Zusammensetzung nach Anspruch 17, **dadurch gekennzeichnet, daß** das Emolliens oder die Emollientien in Mengenanteilen von 10 bis 70 Gew.-%, bezogen auf das Gesamtgewicht der Zusammensetzung, vorliegen.

19. Desodorierende feste Zusammensetzung nach einem der Ansprüche 1 bis 18, **dadurch gekennzeichnet, daß** sie außerdem einen oder mehrere Füllstoffe enthält.

20. Desodorierende feste Zusammensetzung nach Anspruch 19, **dadurch gekennzeichnet, daß** der Füllstoff oder die Füllstoffe unter Talk, Kieselsäure, Stärkederivaten, Tonen oder Acrylatcopolymeren ausgewählt sind.

21. Desodorierende feste Zusammensetzung nach Anspruch 20, **dadurch gekennzeichnet, daß** der Füllstoff oder die Füllstoffe in Mengenanteilen von 0,1 bis 10 Gew.-%, bezogen auf das Gesamtgewicht der Zusammensetzung, vorliegen.

22. Desodorierende feste Zusammensetzung nach einem der Ansprüche 1 bis 21, **dadurch gekennzeichnet, daß** sie ferner weitere Hilfsstoffe wie Parfums oder Farbmittel enthält.

23. Desodorierende feste Zusammensetzung nach einem der Ansprüche 1 bis 22, **dadurch gekennzeichnet, daß** sie als Stift vorliegt.

24. Kosmetische Verwendung einer festen Zusammensetzung nach einem der Ansprüche 1 bis 23, um den Schweißfluß zu vermindern und/oder den unangenehmen Geruch, der bei der Zersetzung des menschlichen Schweißes durch Bakterien entsteht, zu überdecken, zu verbessern oder zu reduzieren.

## Claims

1. Solid deodorant composition which is essentially anhydrous, comprising from 0.01% to 40% by weight of at least one deodorant active principle and, as solidifying agent, a mixture of waxes all having melting points of greater than 80°C comprising:
- at least one synthetic polyethylene wax having a melting point of greater than 80°C, and
- at least one wax of natural origin having a melting point of greater than 80°C.

2. Solid deodorant composition according to Claim 1, **characterized in that** the synthetic wax with a melting point of greater than 80°C is an ethylene homopolymer or a copolymer of ethylene and of another copolymerizable monomer corresponding to the formula
CH₂=CH-R (I)
in which R represents a linear or branched alkyl chain which can be interrupted by mono- or polyoxyalkylene units, an aryl or aralkyl radical, or a -CH₂COOH or -CH₂CH₂OH radical.

3. Solid deodorant composition according to Claim 2, **characterized in that** the ethylene homopolymer or copolymer wax has a weight-average molar mass of between 400 and 1000.

4. Solid deodorant composition according to Claim 3, **characterized in that** the said wax is an ethylene homopolymer having a molar mass of approximately 500.

5. Solid deodorant composition according to any one of Claims 1 to 4, **characterized in that** the natural wax having a melting point of greater than 80°C is chosen from mineral, fossil, animal or vegetable waxes, or hydrogenated oils, fatty esters, fatty alcohols or polyalkoxylated fatty alcohols.

6. Solid deodorant composition according to Claim 5, **characterized in that** the said natural wax is chosen from microcrystalline waxes, ceresin, ozokerite, candelilla wax, carnauba wax, hydrogenated castor oil, hydrogenated palm oil or hydrogenated coconut oil.

7. Solid deodorant composition according to Claim 6, **characterized in that** the natural wax is ozokerite.

8. Solid deodorant composition according to any one of Claims 1 to 7, **characterized in that** the said polyethylene-based wax is present in a proportion of 5 to 20% by weight with respect to the total mass of the composition.

9. Solid deodorant composition according to any one of Claims 1 to 8, **characterized in that** the said natural wax is present in a proportion of 2 to 20% by weight with respect to the total mass of the composition.

10. Solid deodorant composition according to Claim 8 or 9, **characterized in that** the said synthetic polyethylene wax is present in a proportion greater than that of the said natural wax.

11. Solid deodorant composition according to Claim 8 or 9, **characterized in that** the ratio by weight of the said synthetic wax to the said natural wax is between 10/1 and 1/1, preferably between 7/1 and 3/1.

12. Solid deodorant composition according to any of Claims 1 to 11, **characterized in that** the deodorant active principle is present in a proportion of 0.05 to 25% by weight with respect to the total mass of the composition.

13. Solid deodorant composition according to Claims 1 to 12, **characterized in that** the deodorant active principle is chosen from aluminium salts and/or zirconium salts, alum salts, bacteriostatic agents, bactericidal agents, odour-absorbing substances and antioxidizing agents.

14. Solid deodorant composition according to Claim 13, **characterized in that** the deodorant active principle is an aluminium hydroxychloride or an aluminium and zirconium hydroxychloride.

15. Solid deodorant composition according to Claim 14, **characterized in that** the aluminium hydroxychloride or the aluminium and zirconium hydroxychloride is present in a proportion of 2 to 30% by weight, preferably in a proportion of 5 to 25% by weight, with respect to the total mass of the composition.

16. Solid deodorant composition according to any one of Claims 1 to 15, **characterized in that** it additionally comprises one or more emollient agents.

17. Solid deodorant composition according to Claim 16, **characterized in that** the emollient agent or agents are chosen from volatile and non-volatile silicones, polydecenes, fatty acid esters, fatty alcohols, ethoxylated and/or propoxylated surface-active agents, polyalkoxylated esters of glycol or polypropylene glycol (14) butyl ether.

18. Solid deodorant composition according to Claim 17, **characterized in that** the emollient agent or agents are present in a proportion of 10 to 70% by weight with respect to the total mass of the composition.

19. Solid deodorant composition according to any one of Claims 1 to 18, **characterized in that** it additionally comprises one or more fillers.

20. Solid deodorant composition according to Claim 19, **characterized in that** the filler or fillers are chosen from talc, silica, starch derivatives, clay or acrylate copolymers.

21. Solid deodorant composition according to Claim 20, **characterized in that** the filler or fillers are present in a proportion of 0.1 to 10% by weight with respect to the total mass of the composition.

22. Solid deodorant composition according to any one of Claims 1 to 21, **characterized in that** it additionally comprises other adjuvants, such as fragrances or colorants.

23. Solid deodorant composition according to any one of Claims 1 to 22, **characterized in that** it is provided in the form of a stick.

24. Cosmetic use of a solid composition according to any one of Claims 1 to 23 for reducing the flow of sweat and/or for masking, improving or reducing the unpleasant smell resulting from the decomposition of human sweat by bacteria.
